# EUROPEAN PATENT APPLICATION

(11) **EP 0 700 907 A1**
(43) Date of publication of application: **13.03.1996**
(21) Application number: 95113328.9
(22) Date of filing: 24.08.1995
(51) Int. Cl.: C07D 239/34, C07D 239/42

(54) **Process for producing pyrimidine derivative**

(30) Priority: 26.08.1994 JP 201972/94
(71) Applicant: SUMITOMO CHEMICAL COMPANY, LIMITED, Osaka (JP)
(72) Inventor: Uekawa, Toru, Takarazuka-shi, Hyogo (JP); Hirata, Naonori, Sanda-shi, Hyogo (JP); Arai, Kenji, Oita-shi, Oita (JP)
(74) Representative: VOSSIUS & PARTNER

(57) **Abstract**

A process for producing a pyrimidine derivative represented by the following formula: 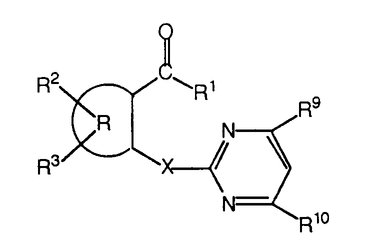
wherein R is an aromatic ring, etc.; X is an oxygen atom, etc.; R¹ , R² and R³ respectively represent a hydrogen atom, etc; and R⁹ and R¹⁰ respectively represent a halogen atom, etc.
which comprises reacting a cyclic compound represented by the following formula: 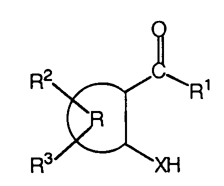
wherein R, X, R¹, R² and R³ are as defined above
with a pyrimidinyltrimethylammonium chloride compound represented by the following formula: 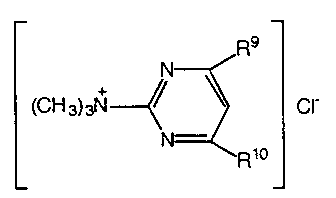
wherein R⁹ and R¹⁰ are as defined above.
The above-mentioned pyrimidine derivative can be easily produced according to the process of the present invention.

## Description

The present invention relates to a process for producing a certain pyrimidine derivative.

As described in Japanese Patent Laid-Open Publication Nos. 4-178371, 6-199761, 6-199810, 62-174059, 63-115870, 64-84, 1-230561, 1-250378, 2-117661, 2-56469, 1-250365, 4-134073, etc., a certain pyrimidine derivative has been known as a useful active ingredient of a herbicide. Thus, it has been desired to develop an advantageous process for producing the pyrimidine derivative.

Therefore, the technical problem of the present application is to provide a process for producing pyrimidine derivatives which have an advantage over the prior art processes. As a result, it has been found that a pyrimidine derivative represented by the formula (1) described below can be obtained under gentle conditions by reacting a cyclic compound represented by the formula (5) described below with a pyrimidinyl trimethylammonium chloride compound represented by the formula (6) described below.

That is, the present invention provides a process for producing a pyrimidine derivative represented by the formula (1): 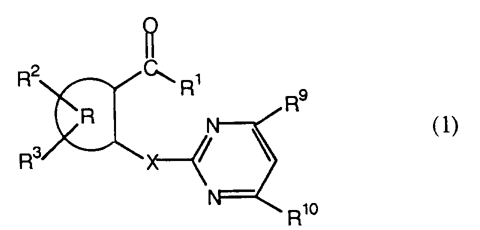
wherein
R is an aromatic ring or an alicyclic ring which may contains a hetero atom;
R¹ is a hydrogen atom, a hydroxyl group, a group of the formula OR⁴.
wherein R⁴ is an alkyl group, an alkenyl group, an alkynyl group , a haloalkyl group, a haloalkenyl group, a haloalkynyl group, a cycloalkyl group, an alkoxyalkyl group or a benzyl group,
or a group represented by the formula (2):

NR⁵R⁶ (2)

wherein R⁵ and R⁶, which are the same or different, represent an alkyl group, an alkenyl group, an alkynyl group, a haloalkyl group, a haloalkenyl group, a haloalkynyl group, a cycloalkyl group or an alkoxyalkyl group,
or a benzyl group which may be substituted with a halogen atom, an alkyl group, an alkoxy group or a haloalkyl group,
or R⁵ and R⁶ bond together at their terminal ends to form an alkylene group,
or a group represented by the formula (3):

N=CR⁵R⁶ (3)

wherein R⁵ and R⁶ are as defined above;
R² and R³, which are the same or different, represent a hydrogen atom, a halogen atom, an alkyl group, an alkenyl group, an alkynyl group, a haloalkyl group, a haloalkenyl group , a haloalkynyl group , an alkoxy group, a haloalkoxy group, a cycloalkyl group, a nitro group, a cyano group or an acyl group,
or an aryl group which may substituted with a halogen atom, an alkyl group, an alkoxy group or a haloalkoxy group,
or an aryloxy group which may be substituted with a halogen atom, an alkyl group, alkoxy group or a haloalkoxy group, or a group represented by the formula (4):

-CR⁷=N-OR⁸ (4)

wherein R⁷ and R⁸ respectively represent a hydrogen atom or an alkyl group;
R⁹ and R¹⁰, which are the same or different, represent a halogen atom , an alkyl group , an alkoxy group or a haloalkoxy group; and X is an oxygen atom or a sulfur atom which comprises reacting a cyclic compound represented by the formula (5): 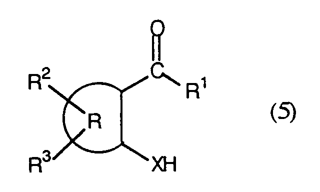
wherein X, R, R¹, R² and R³ are as defined above
with a pyrimidinyltrimethylammonium chloride compound represented by the formula (6) 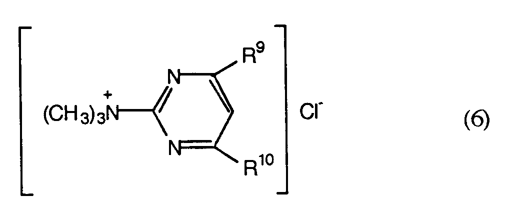
wherein R⁹ and R¹⁰ are as defined above.

The aromatic ring denoted by R means a ring of an aromatic hydrocarbon compound such as benzene and naphthalene or a ring of an aromatic heterocyclic compound such as pyridine, thiophene, pyrrole, benzofuran and quinoline. The alicyclic ring denoted by R may contain a hetero atom such as an oxygen atom, a sulfur atom and a nitrogen atom. Examples of the alicyclic ring include a cyclohexane ring and a tetrahydrofuran ring.

Examples of an alkyl group denoted by R⁴, R⁵ or R⁶ include a C₁-C₆ alkyl group such as a methyl group and an ethyl group. Examples of an alkenyl group denoted by R⁴, R⁵ or R⁶ include a C₃-C₆ alkenyl group such as an allyl group. Examples of an alkynyl group denoted by R⁴, R⁵ or R⁶ include a C₃-C₆ alkynyl group such as a propargyl group. Examples of a haloalkyl group denoted by R⁴, R⁵ or R⁶ include a C₂-C₆ haloalkyl group such as a 2-chlorethyl group and a 2,2,2-trifluoroethyl group. Examples of a haloalkenyl group denoted by R⁴, R⁵ or R⁶ include a C₃-C₆ haloalkenyl group such as a 3-chloroallyl group. Examples of a haloalkynyl group denoted by R⁴, R⁵ or R⁶ include a C₃-C₆ haloalkynyl group such as a 3-chloropropargyl group. Examples of a cycloalkyl group denoted by R⁴, R⁵ or R⁶ include a C₃-C₈ cycloalkyl group such as a cyclopropyl group and a cyclopentyl group. Examples of an alkoxyalkyl group denoted by R⁴, R⁵ or R⁶ include a C₂-C₈ alkoxyalkyl group such as a 2-methoxyethyl group. Examples of a benzyl group denoted by R⁵ or R⁶ include a benzyl group and a 4-chlorobenzyl group. Examples of an alkylene group formed by R⁵ and R⁶ include a tetramethylene group and a penta-methylene group.

Examples of a halogen atom denoted by R², R³, R⁹ or R¹⁰ include a chlorine atom and a fluorine atom. Examples of an alkyl group denoted by R^{2,} R³ ,R⁷, R⁸, R⁹ or R¹⁰ include a C₁-C₆ alkyl group such as a methyl group and an ethyl group. Examples of an alkenyl group denoted by R² or R³ include a C₃-C₆ alkenyl group such as an ally group. Examples of an alkynyl group denoted by R² or R³ include a C₃-C₆ alkynyl group such as a propargyl group. Examples of a haloalkyl group denoted by R² or R³ include a C₁-C₆ haloalkyl group such as a trifluoromethyl group. Examples of a haloalkenyl group denoted by R² or R³ include a C₂-C₆ haloalkenyl group such as a 3-chloroallyl group. Examples of a haloalkynyl group denoted by R² or R³ include a C₂-C₆ haloalkynyl group such as a 3-chloropropargyl group. Examples of a cycloalkyl group denoted by R² or R³ include a C₃-C₈ cycloalkyl group such as a cyclopropyl group and a cyclopentyl group. Examples of an aryl group denoted by R² or R³ include a phenyl group. Examples of an aryloxy group denoted by R² or R³ include a phenoxy group. Examples of an alkoxy group denoted by R², R³, R⁹ or R¹⁰ include a C₁-C₆ alkoxy group such as a methoxy group and an ethoxy group. Examples of an haloalkoxy group denoted by R² or R³ include a C₁-C₆ haloalkoxy group such as a trifluoromethoxy group and a difluoromethoxy group. Examples of an haloalkoxy group denoted by R⁹ or R¹⁰ include a C₁-C₆ haloalkoxy group such as a trifluoromethoxy group, a difluoro-methoxy group and a 2,2,2-trifluoroethoxy group. Examples of an acyl group denoted by R² or R³ include an acetyl group.

In the production process of the present invention, the reaction is normally conducted with or without a solvent in the presence or absence of a base, preferably in the presence of the base. The reaction temperature is normally within the range of -20°C to the boiling point of the solvent or to 80°C, preferably 0 to 50°C. The reaction time is normally within the range of 5 minutes to 24 hours. The amount of the cyclic compound represented by the formula (5) to be subjected to the reaction and the base to be optionally used are normally within the range of 0.5 to 1.5 mol and within the range of 1 to 20 mol, respectively, based on 1 mol of the pyrimidinyl trimethylammonium chloride represented by the formula (6).

Examples of the solvent to be optionally used include aliphatic hydrocarbons such as hexane, heptane, ligroin and petroleum ether; aromatic hydrocarbons such as benzene, toluene and xylene; halogenated hydrocarbons such as chloroform, carbon tetrachloride, dichloroethane, chlorobenzene and dichlorobenzene; ethers such as diethyl ether, diisopropyl ether, dioxane, tetrahydrofuran and diethylene glycol methyl ether; ketones such as acetone, methyl ethyl ketone, methyl isobutyl ketone, isophorone and cyclohexanone; alcohols such as methanol, ethanol, isopropanol, t-butanol, octanol, cyclohexanol, methyl cellosolve, diethylene glycol and glycerin; esters such as ethyl formate, ethyl acetate, butyl acetate and diethyl carbonate; nitrated substances such as nitroethane and nitro-benzene; nitriles such as acetonitrile and isobutyronitrile; tertiary amines such as pyridine, triethylamine, N,N-diethylaniline, tributylamine and N-methylmorpholine; amides such as formamide, N,N-dimethylformamide and acetamide; sulfur compounds such as dimethyl sulfoxide and sulfolane; liquid ammonia; water; and a mixture thereof.

Examples of the base to be optionally used include organic bases such as pyridine, trimethylamine, triethylamine and N,N-diethylaniline; inorganic bases such as sodium hydroxide, potassium hydroxide, sodium carbonate, potassium carbonate and sodium hydride; alkali metal alkoxides such as sodium methoxide and sodium ethoxide; metal fluorides such as potassium fluoride and cesium fluoride; quaternary ammonium salts such as tetrabutylammonium chloride and tetrabutyl-ammonium bromide; and a mixture thereof.

In the production process of the present invention, the reaction may be optionally conducted in the presence of phase transfer catalysts such as quaternary ammonium salts (e.g. tetrabutylammonium chloride, tetrabutylammonium bromide, etc.), or crown ethers (e.g. 18-crown-6, etc.).

After the completion of the reaction, water can be added to the reaction solution, and the reaction solution can be subjected to a normal post-reaction treatment such as organic solvent extraction and concentration and, if necessary, it is purified by an operation such as chromatography, recrystallization and distillation to isolate the objective pyrimidine derivative represented by the formula (1).

According to the production process of the present invention, there can be easily produced a pyrimidine compound wherein R¹ in the formula (1) is a comparatively unstable group, i.e. a group of ONR⁵R⁶ or ON=CR⁵R⁶ (wherein R⁵ and R⁶ are as defined above)
Examples of the pyrimidine derivative represented by the formula (1) which can be produced according to the production process of the present invention include the following.
2-Chloro-6-(4,6-dimethoxypyrimidin-2-yl)oxymethyl benzoate
2-Chloro-6-(4,6-dimethoxypyrimidin-2-yl)oxyethyl benzoate
2-(4,6-Dimethoxypyrimidin-2-yl)oxy-1-(N,N-diethylaminooxy-carbonyl) naphthalene
2-(4,6-Dimethoxypyrimidin-2-yl)oxy-1-(N,N-dimethylaminooxycarbonyl) naphthalene
3-(4,6-Dimethoxypyrimidin-2-yl)oxy-1-(N,N-diethylaminooxycarbonyl) pyridine
3-(4,6-Dimethoxypyrimidin-2-yl)oxy-(N,N-dimethylaminooxycarbonyl) pyridine
4,6-Dimethoxy-2-{3-chloro-2-(N,N-diethylaminooxycarbonyl) phenoxy}pyrimidine
4,6-Dimethoxy-2-{3-chloro-2-(N,N-dimethylaminooxycarbonyl) phenoxy}pyrimidine
The cyclic compound represented by the formula (5) as another starting compound in the production process of the present invention can be produced, for example, according to the process described in J. Org. Chem., 27, 3551 (1962), Chem. Pharm, Bull., 31, 407 (1983), Pharmaceutical Journal, 99, 657 (1979), Japanese Laid-Open Patent Publication No. 4-178371, etc.

The pyrimidinyltrimethyl ammonium chloride compound represented by the formula (6) as another starting compound in the production process of the present invention can be produced, for example, according to the process described in Japanese Laid-Open Patent Publication Nos. 2-115174 and 58-206572, Liebigs Ann. Chem., 333 (1981), etc. The compound of formula (6) wherein R⁹ and R¹⁰ are methoxy groups can be produced by reacting 2-chloro-4,6-dimethoxypyrimidine with trimethylamine (hereinafter this process is referred to as production process "a").

In production process "a", the reaction is normally conducted with or without a solvent. The reaction temperature is normally within the range of -50°C to the boiling point of the solvent or to 50°C, preferably 0 to 30°C. The reaction time is normally within the range of 5 minutes to 48 hours. The amount of trimethylamine to be subjected to the reaction is normally within the range of 1 mol or more, e.g. 1 to 10 mol, based on 1 mol of 2-chloro-4,6-dimethoxypyrimidine. Examples of the solvent to be optionally used include aliphatic hydrocarbons such as hexane, heptane, ligroin and petroleum ether; aromatic hydrocarbons such as benzene, toluene and xylene; halogenated hydrocarbons such as chloroform, carbon tetrachloride, dichloroethane, chlorobenzene and dichlorobenzene; ethers such as diethyl ether, diisopropyl ether, dioxane, tetrahydrofuran and diethylene glycol methyl ether; ketones such as acetone, methyl ethyl ketone, methyl isobutyl ketone and isophorone, cyclohexanone; alcohols such as methanol, ethanol, isopropanol, t-butanol, octanol, cyclohexanol, methyl cellosolve, diethylene glycol and glycerin; esters such as ethyl formate, ethyl acetate, butyl acetate and diethyl carbonate; nitrated substance such as nitroethane and nitrobenzene; nitriles such as acetonitrile and isobutyronitrile; amides such as formamide, N,N-dimethylformamide and acetamide; sulfur compounds such as dimethyl sulfoxide and sulfolane; water; and a mixture thereof. After the completion of the reaction, the reaction solution can be subjected to a normal post-reaction treatment such as filtration, concentration, etc. to isolate (4,6-dimethoxy-2-pyrimidinyl)-trimethylammonium chloride.

In the production process of the present invention, an isolated pyrimidinyltrimethyl-ammonium chloride compound represented by the formula (6) can be used, but the compound not isolated from the crude reaction product may also be used.

### Example 1

84 mg (2.1 mmol) of 60% oily sodium hydride was suspended in 15 ml of N,N-dimethylformamide, and then a solution prepared by dissolving 0.37 g (2.0 mmol) of 6-chloro methyl salicylate in 2 ml of N,N-dimethylformamide was added dropwise at 0°C. After the completion of dropwise addition , the mixture was stirred at room temperature for 30 minutes and 0.49 g (2.1 mmol) of (4,6-dimethoxy-2-pyrimidinyl) trimethylammonium chloride was added thereto. After stirring at room temperature for one hour, the reaction solution was poured into water and was subjected to an extraction with ethyl acetate. The organic layer was dried with anhydrous magnesium sulfate. The solvent was distilled off under reduced pressure and the resulting residue was subjected to silica gel column chromatography to obtain 0.64 g of 2-chloro-6-(4,6-dimethoxypyrimidin-2-yl)oxy methyl benzoate as a pale yellow liquid (yield: 98%).
¹H-NMR (CDCl₃/TMS) δ (ppm): 3.77 (s, 9H), 5.71 (s, 1H), 6.97-7.35 (m, 3H).

### Example 2

84 mg (2.1 mmol) of 60% oily sodium hydride was suspended in 15 ml of N,N-dimethylformamide, and then a solution prepared by dissolving 0.52 g (2.0 mmol) of 2-hydroxy-1-(N,N-diethylamino-oxycarbonyl)naphthalene in 2 ml of N,N-dimethylformamide was added dropwise at 0°C. After the completion of dropwise addtion, the mixture was stirred at room temperature and 0.49 g (2.1 mmol) of (4,6-dimethoxy-2-pyrimidinyl) trimethyl-ammonium chloride was added. After stirring at room temperature for one hour, the reaction solution was poured into water and subjected to an extraction with ethyl acetate. The organic layer was dried over anhydrous magnesium sulfate. The solvent was silica gel column chromatography to obtain 0.74 g of 2-(4,6-dimethoxypyrimidin-2-yl)oxy-1-(N,N-diethylaminooxycarbonyl)naphthalene as a colorless crystal (yield: 93%).
Melting point: 92.8°C

### Example 3

96 mg (2.4 mmol) of 60% oily sodium hydride was suspended in 15 ml of N,N-dimethylformamide, and then a solution prepared by dissolving 0.48 g (2.3 mmol) of 3-hydroxy-2-(N,N-diethylaminooxycarbonyl)pyridine in 2 ml of N,N-dimethyl-formamide was added dropwise at 0°C. After the completion of dropwise addition, the mixture was stirred at room temperature for 30 minutes and 0.56 g (2.4 mmol) of (4,6-dimethoxy-2-pyrimidinyl)trimethylammonium chloride was added. After stirring at room temperature for one hour, the reaction solution was poured into water and was subjected to an extraction with ethyl acetate. The organic layer was dried over anhydrous magnesium sulfate . The solvent was distilled off under reduced pressure and the resulting residue was subjected to silica gel column chromatography to obtain 0.54 g of 3-(4,6-dimethoxy-pyrimidin-2-yl)oxy-(N,N-diethylaminooxycarbonyl)pyridine as a colorless crystal (yield: 67%).
Melting point: 73.2°C

### Example 4

To a solution prepared by dissolving 1.18 g (0.02 mol) of trimethylamine in 20 ml of methyl isobutyl ketone, 0.35 g (2.0 mmol) of 2-chloro-4,6-dimethoxypyrimidine was added and the mixture was stirred to produce (4,6-dimethoxy-2-pyrimidinyl)-trimethylammonium chloride in the reaction system. Then, 0.49 g (2 mmol) of 3-chloro-2-(N,N-diethylaminooxycarbonyl)phenol was added thereto. After stirring at room temperature for 3 hours, the reaction solution was poured into water and was subjected to an extraction with ethyl acetate. The organic layer was dried over anhydrous magnesium sulfate . The solvent was distilled off under reduced pressure and the resulting residue was subjected to silica gel column chromatography to obtain 0.61 g of 4,6-dimethoxy-2-{3-chloro-2-(N,N-diethylaminooxycarbonyl)-phenoxy}pyrimidine as a colorless crystal (yield: 80%).
Melting point: 107.4°C

### Example 5

To a solution prepared by dissolving 0.49 g (2 mmol) of 3-chloro-2-(N,N-diethylaminooxycarbonyl)phenol in 10 ml of methyl isobutyl ketone, 0.28 g (2 mmol) potassium carbonate was added and the mixture was stirred at room temperature for 10 minutes. Then, 0.49 g (2.1 mmol) of (4,6-dimethoxy-2-pyrimidinyl)trimethylammonium chloride was added thereto. After stirring at room temperature for 5 hours, the reaction solution was poured into water and was subjected to an extraction with ethyl acetate. The organic layer was dried over anhydrous magnesium sulfate . The solvent was distilled off under reduced pressure and the resulting residue was subjected to silica gel column chromatography to obtain 0.51 g of 4,6-dimethoxy-2-{3-chloro-2-(N,N-diethylaminooxycarbonyl)-phenoxy}pyrimidine as a colorless crystal (yield: 67%).

Hereinafter, Production Example of (4,6-dimethoxy-2-pyrimidinyl)trimethylammonium chloride will be described.

### Production Example

17.7 g (0.300 mol) of trimethylamine was dissolved in 200 ml of benzene and 2-chloro-4,6-dimethoxypyrimidine [described in Pesticide Production Technique 9, 17 (1963)] (5.24 g, 30.0 mmol) was added thereto. Then, the mixture was stirred at room temperature for 8 hours. The precipitated solid was filtered and the resulting solid was dried under reduced pressure to obtain 5.60 g of (4,6-dimethoxy-2-pyrimidinyl)trimethylammonium chloride as a white powdered solid.
Melting point: 150.3°C
¹H-NMR (CDCl₃/TMS) δ (ppm): 3.96 (s, 9H), 4.04 (s, 6H), 6.17 (s, 1H)

## Claims

1. A process for producing a pyrimidine derivative represented by the formula (1): 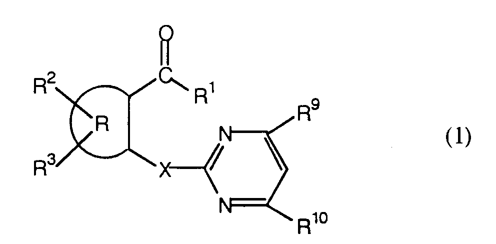 wherein
R is an aromatic ring or an alicyclic ring which may contains a hetero atom;
R¹ is a hydrogen atom, a hydroxyl group, a group of the formula OR⁴.
wherein R⁴ is an alkyl group, an alkenyl group, an alkynyl group, a haloalkyl group, a haloalkenyl group, a haloalkynyl group, a cycloalkyl group, an alkoxyalkyl group or a benzyl group,
or a group represented by the formula (2):
NR⁵R⁶ (2)
wherein R⁵ and R⁶, which are the same or different, represent an alkyl group, an alkenyl group, an alkynyl group, a haloalkyl group, a haloalkenyl group, a haloalkynyl group, a cycloalkyl group or an alkoxyalkyl group,
or a benzyl group which may be substituted with a halogen atom, an alkyl group, an alkoxy group or a haloalkyl group,
or R⁵ and R⁶ bond together at their terminal ends to form an alkylene group,
or a group represented by the formula (3):
N=CR⁵R⁶ (3)
wherein R⁵ and R⁶ are as defined above;
R² and R³, which are the same or different, represent a hydrogen atom, a halogen atom, an alkyl group, an alkenyl group, an alkynyl group , a haloalkyl group, a haloalkenyl group , a haloalkynyl group , an alkoxy group, a haloalkoxy group, a cycloalkyl group, a nitro group, a cyano group or an acyl group,
or an aryl group which may substituted with a halogen atom, an alkyl group, an alkoxy group or a haloalkoxy group,
or an aryloxy group which may be substituted with a halogen atom, an alkyl group, alkoxy group or a haloalkoxy group, or a group represented by the formula (4):
-CR⁷=N-OR⁸ (4)
wherein R⁷ and R⁸ respectively represent a hydrogen atom or an alkyl group;
R⁹ and R¹⁰, which are the same or different, represent a halogen atom , an alkyl group , an alkoxy group or a haloalkoxy group; and X is an oxygen atom or a sulfur atom
which comprises reacting a cyclic compound represented by the formula (5): 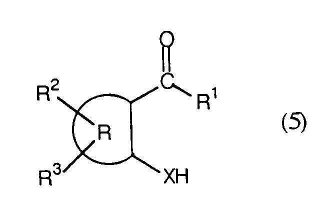 wherein X, R, R¹, R² and R³ are as defined above
with a pyrimidinyltrimethylammonium chloride compound represented by the formula (6): 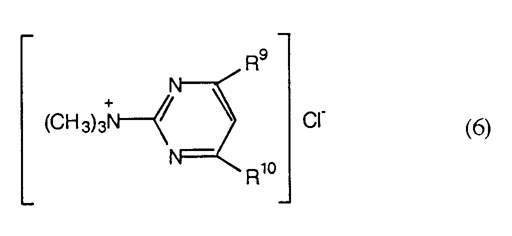 wherein R⁹ and R¹⁰ are as defined above.

2. The process according to claim 1, wherein R is a benzene, naphthalene or pyridine ring.

3. The process according to claim 1 or 2, wherein R⁹ and R¹⁰ are dimethoxy groups.

4. The process according to any of claims 1 to 3, wherein R¹ is a group of the formula ONR⁵R⁶ or ON=CR⁵R⁶.

5. The process according to any of claims 1 to 4, wherein X is an oxygen atom.

6. The process according to any of claims 1 to 5, wherein R² and R³, which are the same or different, each represent a hydrogen atom, a halogen atom, an alkyl group, a haloalkyl group or an alkoxy group, or a phenyl group which may be substituted with a halogen atom, an alkyl group, an alkoxy group or a haloalkoxy group.

7. (4,6-Dimethoxy-2-pyrimidinyl)trimethylammonium chloride.
